# EUROPEAN PATENT APPLICATION

(11) **EP 1 705 168 A1**
(43) Date of publication of application: **27.09.2006**
(21) Application number: 05006117.5
(22) Date of filing: 21.03.2005
(51) Int. Cl.: C07C 17/14, C07C 22/04

(54) **Improved process for side-chain bromination of alkyl-benzenes**

(71) Applicant: Helm AG, 20097 Hamburg (DE)
(72) Inventor: Schulze, Michael, DE-20073 Hamburg (DE); Stritzke, Katja Dr., DE-20255 Hamburg (DE)
(74) Representative: UEXKÜLL & STOLBERG

(57) **Abstract**

A process for the side-chain bromination of alkylbenzenes according to Formula (I) 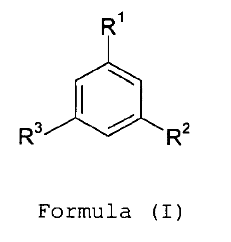 wherein R¹ is C₁-C₆-alkyl; R² is H or C₁-C₆-alkyl which can be unsubstituted or substituted by one or more cyano groups; R³ is H or C₁-C₆-alkyl which can be unsubstituted or substituted by one or more cyano groups; comprising the steps of (1) dissolving the compound of Formula (I) in a solvent which is a nonaromatic non-halogenated hydrocarbon which can be substituted by one or more cyano groups; (2) adding a bromination agent selected from the group consisting of Br₂ and N-bromoimides to the solution; and optionally (3) agitating the mixture at a temperature within the range of from room temperature to reflux temperature.

## Description

This invention relates to a process for the side-chain bromination of alkylbenzenes and in particular of alkylbenzenes forming intermediates in the synthesis of anastrozole.

The reagent of choice for side-chain bromination of alkylbenzenes is N-bromosuccinimide. This reagent is commonly used in combination with the solvent carbon tetrachloride. Other solvents are reported to result in side reactions such as ring substitution (Organikum, VEB Deutscher Verlag der Wissenschaften, Berlin 1981). A major drawback of carbon tetrachloride is, that it is both toxic and carcinogenic and that it exhibits ozone-layer damaging properties.

Mitchell et al., Can. J. Chem. Vol. 52, 1974, 3054, reported a yield of 60 to 80 % for the formation of 3,5-bis-(bromomethyl)toluene by bromination of mesitylene with N-bromosuccinimide in refluxing carbon tetrachloride.

Vögtle et al., Chem. Ber. 106, 717-718 (1973), obtained 1,3,5-tris-(bromomethyl)benzol in 23 % yield upon photocatalysed bromination of mesitylene with NBS in boiling carbon tetrachloride.

C. Djerassi, Chem. Rev. 43, 271 (1948), describes the use of other solvents than carbon tetrachloride for brominations with N-bromoimides. Benzene was found to be advantageous when used in combination with N-bromosuccinimide. Petroleum ether and heptane are said not to offer any obvious advantages whereas chloroform is reported to give excellent results particularly in large-scale runs. Carbon disulfide gave only poor results in the bromination of toluene. Acetic anhydride, ethyl acetate, dioxane and tri-n-butylamine are mentioned as possible solvents, but N-bromosuccinimide is said to be unstable therein. It is noted that the reaction may be carried out in an excess of the reactant without the addition of a solvent.

Amijs et al., Green Chemistry, 2003, 5, 470-474, describe the use of methyl acetate as solvent for N-bromosuccinimide side-chain bromination reactions of halide-substituted alkyl arenes. Product yields are said to be comparable to those obtained in carbon tetra chloride.

Offermann and Vögtle, Synthesis, 1977, 4, 272, reported benzylic brominations of arenes with N-bromosuccinimide in non-chlorinated solvents, such as methyl formate and methyl acetate. Selectivity of bromine attack was fond to depend on the solvent employed. The influence of the solvent was ascribed to its electron donating/accepting properties and its polarizability.

In a later study, Offermann and Vögtle, Angew. Chem. 92 (1980) Nr. 6, 471, the list of solvents for this reaction was extended to *t*BuCO₂Me, CH₂Cl₂, CHCl₃, C₆H₆ and CS₂. Selectivity for bromination of the aromatic ring was found to be high for solvents having a low refraction index. The authors recommend the use of methyl formate and methyl acetate since these solvents combine a low refraction index with low application temperature. However, it was also found that selective solvents favour ring bromination of electron rich arenes.

Mataka et al., Bull. Chem. Soc. Jpn., 67, 1113-1119 (1994), describe the tri- and dibromination of methyl-, dimethyl-, and trimethylbenzenes with N-bromosuccinimide in carbon tetrachloride or benzene. The reaction in benzene is said to be slower than the reaction in carbon tetrachloride. Bromination of 1,3,5-trimethylbenzene in carbon tetrachloride gave 1,3,5-tris(tribromomethyl)benzene in 79 % yield, while bromination in benzene gave this product in only 51 % yield together with 24 % of an octabrominated by-product. Bromination of the methyl groups was affected by the presence of ortho substituents on the aromatic ring.

Diez-Barra et al., J. Org. Chem. 2001, 66, 5664-5670, describe the bromination of 1-bromo-3,5-dimethylbenzene and 1,3-dibromo-5-methylbenzene with N-bromosuccinimide in refluxing acetonitrile.

Besides N-bromosuccinimide molecular bromine can be used for side-chain brominations of alkylated benzene derivatives.

Ried and Königstein, Chem. Ber., 92, 2532 (1959), describe the synthesis of 1-methyl-3,5-bis-bromomethyl benzene by reaction of 1,3,5-trimethylbenzene (mesitylene) by photochemical bromination with bromine. Bromination is performed in boiling mesitylene in the absence of additional solvent. No yield for this reaction is reported.

The iodine bromide catalysed reaction between mesitylene and bromine in carbon tetrachloride was reported to result mainly in ring-bromination and only a negligible amount of side-chain bromination (Blake and Keefer, J. Am. Chem. Soc., 77, 3707 (1955).

Mestres and Palenzuela, Green Chem., 2002, 4, 314, reported the benzylic bromination of 4-benzoyloxytoluene using a combined bromine-hydrogen peroxide system in non-chlorinated solvents. Hexane was found not to be convenient while benzene gave fairly good results. Esters, such as ethyl acetate, isopropyl acetate, methyl isobutyrate, and *tert*-butyl methyl ether gave poor conversions. Methyl pivalate gave a satisfactory bromination yield, although the formation of side products was observed.

Illuminati and Marino J. Am. Chem. Soc., 78, 4975 (1956), determined the bromination rates of halogen-substituted mesitylenes, isodurenes and durenes in nitromethane, acetic acid and mixtures thereof for the bromination with molecular bromine.

Keefer and Andrews, J. Am. Chem. Soc., 78, 3637 (1956), obtained ring substituted 2-bromomesitylene in a yield of 40 % upon bromination of mesitylene in 90 % aqueous acetic acid with bromine.

Sampey et al., J. Am. Chem. Soc., 62, 1839 (1940), describe the side-chain bromination of substituted toluenes by reaction with bromine in chloroform. Since bromination of this solvent was observed, carbon disulfide and carbon tetrachloride were used as alternative solvents. Carbon tetrachloride showed no bromination when heated with bromine and gave faster rates of bromination than carbon disulfide.

The side-chain bromination of alkylbenzenes is of particular importance in the synthesis of the pharmaceutically active compound anastrozole (α,α,α',α'-tetramethyl-5-(1H-1,2,4-triazol-1-arylmethyl)-1,3-benzenediacetonitrile) which is a potent and selective non-steroidal aromatase inhibitor. Aromatase is an enzyme which effects aromatisation of ring A in the metabolic formation of steroid hormones. Various cancers, such as breast cancer, are dependent upon circulating steroid hormones which have an aromatic ring A. Such cancers can be treated by inhibiting the aromatisation of steroid ring A. The synthesis of anastrozole involves two bromination steps.

According to EP 0 296 749 anastrozole is prepared by treating 3,5-bis-bromomethyltoluene with potassium cyanide to give 5-methyl-1,3-benzenediacetonitrile. Treatment of this compound with sodium hydride and iodomethane gives α,α,α',α'-tetramethyl-5-methyl-1,3-benzene-diacetonitrile, which is subsequently treated with N-bromosuccinimide in carbon tetrachloride. The brominated compound is then reacted with sodium triazole in dimethylformamide to give anastrozole.

None of the above methods for side-chain bromination of alkylated benzenes is completely satisfying. The use of chlorinated solvents is undesirable for environmental reasons. The same applies to aromatic solvents such as benzene. The use of esters such as methyl acetate or methyl formate is disadvantageous because they enhance the formation of side-products and impair the stability of the bromination agent.

It is the object of the present invention to provide a method for the side-chain bromination of alkylbenzenes which does not reauire the use of the above solvents and which allows the preparation of brominated products in good yields and with less side-reactions. Another object is to provide an improved process for the synthesis of anastrozole.

This object is achieved by a process for the side-chain bromination of alkylbenzenes according to Formula (I) 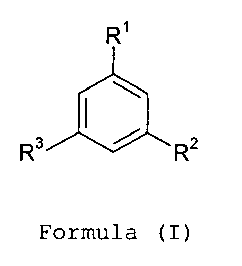 wherein
- R¹: is C₁-C₆-alkyl;
- R²: is H or C₁-C₆-alkyl which can be unsubstituted or substituted by one or more cyano groups;
- R³: is H or C₁-C₆-alkyl which can be unsubstituted or substituted by one or more cyano groups;
which comprises the steps of
(1) dissolving the compound of Formula (I) in a solvent which is a non-aromatic non-halogenated hydrocarbon which can be substituted by one or more cyano groups;
(2) adding a bromination agent selected from the group consisting of Br₂ and N-bromoimides to the solution;
(3) and optionally agitating the mixture at a temperature within the range of from room temperature to reflux temperature.

The product of the process can be isolated and purified by standard methods such as washing of the reaction mixture, extraction, column chromatography and/or crystallisation.

It was surprisingly found by the present inventors that alkylbenzenes according to formula (I) can be brominated with molecular bromine and N-bromoimides in good yields and with the formation of less side products if the reaction is performed in the solvents defined above.

The alkylbenzenes of formula (I) are hydrocarbons, i.e. compounds consisting of hydrogen and carbon atoms, which can be substituted by one or more cyano groups. Apart from the nitrogen(s) of the optional cyano group(s) no other heteroatoms are present; these compounds do not contain halogen or oxygen atoms. Preferably, R¹ is C₁-C₃-alkyl, R² is C₁-C₃-alkyl, and R³ is C₁-C₃-alkyl. R² and/or R³ can be unsubstituted or substituted by one or more cyano groups. It is particularly preferred that R² and/or R³ are unsubstituted or substituted by one cyano group. Most preferably the compound of formula (I) is 1,3,5-trimethylbenzene (mesitylene) or α,α,α',α'-tetramethyl-5-methyl-1,3-benzene-diacetonitrile.

The solvents used in step (1) of the bromination are hydrocarbons, i.e. compounds consisting of hydrogen and carbon atoms. They may be unsubstituted or substituted by one or more, preferably one cyano group. Apart from the nitrogen(s) of the optional cyano group(s) no other heteroatoms are present. In general, the solvent should be as apolar as possible.

These solvents are liquid at room temperature and preferably have a boiling point of 35 to 130 °C, more preferably 50 to 100 °C and in particular of 75 to 85 °C. Preferred solvents are cyclohexane and acetonitrile.

According to a preferred embodiment of the invention a radical initiator, preferably a peroxide catalyst is added to the reaction mixture, more preferably azobisisobutyronitrile (AIBN) or dibenzoyl peroxide.

Preferred N-bromoimides are N-bromophthalimide, N-bromoacetamide and in particular N-bromosuccinimide (NBS).

In the process of the present invention the bromination agent is added to a solution of a compound of Formula (I) in a solvent as defined above. During the addition of the bromination agent the mixture is preferably kept at a temperature within the range of from room temperature to reflux temperature. After the addition of the bromination agent is completed the reaction mixture is preferably stirred for an additional time period of 1 minute to 3 hours, more preferably 0.5 to 1 hour at the temperature indicated above.

The solvent is preferably used in such an amount that the concentration of the compound of Formula (I) in the solvent is within a range of 0.1 to 2.5 mol/l, more preferably 0.2 to 1.5 mol/l and most preferably 0.3 to 1.0 mol/l.

The bromination agent is preferably used in a concentration of 1.0 to 1.5 meq per bromination site, more preferably 1.0 to 1.2 meq, and most preferably in a concentration of 1.05 meq per bromination site.

The initiator is preferably used in a concentration of 0.015 meq.

For unsubstituted compounds according to formula (I) and in particular for compounds wherein R¹ is C₁-C₃-alkyl, R² is C₁-C₃₋alkyl, and R³ is C₁-C₃-alkyl, N-bromoimides, preferably NBS, or molecular bromine are advantageously used as the bromination agent. The preferred solvent for this group of compounds is cyclohexane. Thus, bromination of unsubstituted compounds of formula (I) is advantageously achieved by NBS or molecular bromine in cyclohexane. The concentration of these educts in the solvent is preferably within a range of 0.1 to 1 mol/l, more preferably 0.2 to 0.6 mol/l and most preferably about 0.3 mol/l.

Surprisingly, HPLC analysis of the bromination reaction of mesitylene with NBS in cyclohexane showed a significant increase of brominated products when compared to the same reaction in carbon tetrachloride. 3,5-Bisbromomethyl toluene was formed in an amount of about 60 % and the tribromo product 1,3,5-tribromomethyl benzene in an amount of about 21 %. In contrast, bromination of mesitylene with NBS in carbon tetrachloride gave only about 39 % of 3,5-bisbromomethyl toluene and 21 % of 1,3,5-tribromomethyl benzene while about 40 % of side products were formed.

HPLC analysis of the bromination reaction of mesitylene with Br₂ in cyclohexane revealed about 55 % of 3,5-bisbromomethyl toluene and about 22 % of 1,3,5-tribromomethyl benzene.

For compounds bearing a cyano group, in particular for compounds wherein R¹ is C₁-C₃-alkyl, R² is C₁-C₃-alkyl which is substituted by one cyano group, and R³ is C₁-C₃-alkyl which is substituted by one cyano group, bromination is preferably achieved by use of NBS as the bromination agent. The preferred solvents for cyano-substituted compounds according to formula (I) are cyclohexane and in particular acetonitrile, and the preferred bromination system comprises the use of NBS in combination with cyclohexane or acetonitrile. The concentration of these educts in the solvent is preferably within a range of 0.3 to 2.5 mol/l, more preferably 0.5 to 1.5 mol/l and most preferably about 0.8 mol/l.

Bromination of α,α,α',α'-tetramethyl-5-methyl-1,3-benzene-diacetonitrile with NBS in acetonitrile gave about 81 % of the desired product α,α,α',α'-tetramethyl-5-bromomethyl-1,3-benzene-diacetonitrile, as determined by HPLC, while the corresponding reaction in carbon tetrachloride gave only about 70 % of this product.

The process of the present invention is particularly suitable in the synthesis of anastrozole. This compound is accessible by subjecting 1,3,5-tribomobenzene to the process as defined above. Next the bromine residues of the reaction product 3,5-bisbromomethyl toluene are substituted by cyano groups, preferably by reaction with alkali cyanide, preferably NaCN or KCN, and the cyano substituted products is again subjected to the process as defined above. The alkyl residues of the cyano substituted product can be alkylated, preferably in α-position to the cyano groups, before proceeding to the second bromination reaction. Alkylation can be achieved by reaction with iodoalkanes such as C₁-C₃-iodoalkones and in particular methyliodide.

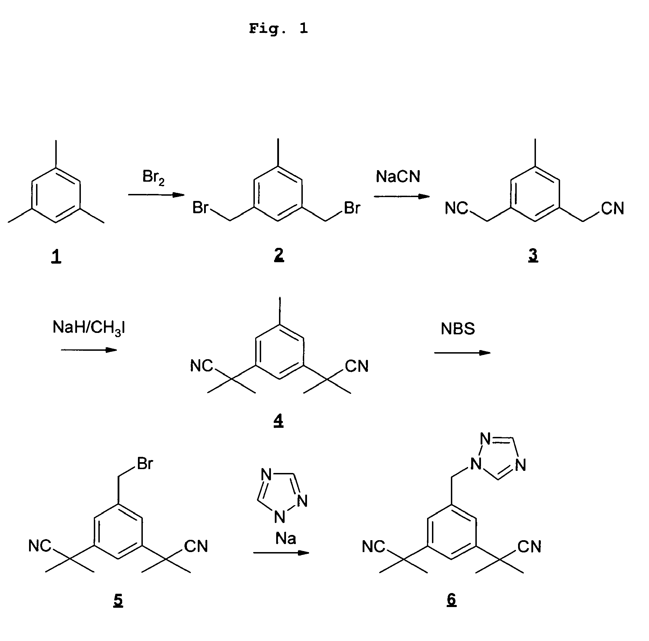

A particularly preferred process for the synthesis of anastrozole is shown in Figure 1. 1,3,5-Tribomobenzene **1** is converted to 3,5-bisbromomethyl toluene **2** by bromination with molecular Br₂ in cyclohexane. The product can be isolated and purified by standard methods known to a person skilled in the art. Advantageously, the product is isolated by washing the reaction mixture, preferably with water, extracting the organic phase, preferably with ethyl acetate, followed by vacuum distillation. 3,5-Bisbromomethyl toluene **2** can be crystallised from ethanol. The bromine residues of compound **2** are preferably substituted by cyano groups by reaction with NaCN, preferably in CH₂Cl₂/water, to obtain 5-methyl-1,3-benzenediacetonitrile **3.** This product is again isolated by washing and extraction of the reaction mixture. 5-methyl-1,3-benzenediacetonitrile **3** can be crystallised from isopropanol. Compound **3** is then methylated in α-position to the cyano groups, preferably by reaction with NaH and methyl iodide to give α,α,α',α'-tetramethyl-5-methyl-1,3-benzene-diacetonitrile **4.** This product is isolated by washing and extraction and can be crystallised from isopropanol. Bromination of compound **4** with NBS in acetonitrile gives α,α,α',α'-tetramethyl-5-bromomethyl-1,3-benzene-diacetonitrile **5.** Finally, compound **5** is converted to anastrozole **6** by reaction with 1,2,4-triazole sodium.

According to the process of the invention anastrozole can be produced without the requirement of toxic solvents. Furthermore, intermediates **2** and **5** are surprisingly obtained in improved yields when compared to the reaction in carbon tetrachloride.

The process of the invention is not restricted to the synthesis of anastrozole. Other side-chain brominated alkylbenzenes which are suitable e.g. as intermediates in the synthesis of numerous other compounds can be obtained in good yields as well. Due to the reduced toxicity of the solvents used, the process of the invention is particularly suitable for the synthesis of pharmaceutically active compounds and intermediates thereof.
In the following the invention will be further explained by the use of examples.

### Examples

### Example 1: 3,5-bisbromomethyl toluene (2)

Mesitylene 1 (6 kg; 50 mol) was diluted in a 25-fold excess of cyclohexane (150 1). The solution was heated to about 70 °C. NBS (17.7 kg, 105 mol, 2.1 meq) and AIBN (0.23 kg, 0.75 mol, 0.015 meq) were added stepwise to the hot solution. After the final addition, the mixture was heated at reflux for 30 minutes, and then cooled to room temperature. The reaction mixture was analysed by HPLC (column: RP-18, 4 x 250 mm, 5 µm (Merck, Lot L 552433); detection: UV = 265 nm; flow rate: 1 ml/min; mobile phase: acetonitrile/water - 2:1), the results of the HPLC analysis are shown in Table 1. For isolating the product in pure form, the mixture was filtered, and the filtrate was washed with 100 1 of water three times. The organic layer was concentrated to dryness under reduced pressure. The residue was fractionally distilled under reduced pressure (7~10torr, 136~156°C). The fraction collected was then crystallised from EtOH/hexane to obtain the purified 3,5-bisbromomethyl toluene **2.** Yield: 39 %.

The reaction was repeated again but this time using carbon tetrachloride as the solvent. The reaction mixture was analysed by HPLC as described above. The results are also shown in Table 1. 3,5-Bisbromomethyl toluene **2** was isolated in a yield of 24 %.

**Table 1 Bromination of Mesitylene**

| **solvent/ bromination agent** | **3,5-bisbromomethyl toluene 2 [%]**^{**1)**} | **1,3,5-tribromomethyl benzene [%]**^{**1)**} | **side products [%]**^{**1)**} |
|---|---|---|---|
| CCl₄/ NBS²⁾ | 38.5 | 21.4 | 40.1 |
| Cyclohexane/ NBS | 60.3 | 20.9 | 18.8 |
| Cyclohexane/ Br₂³⁾ | 55.1 | 22.3 | 22.6 |

| | | | |
|---|---|---|---|
| 1) determined by HPLC | | | |
| 2) comparative example | | | |
| 3) Example 6 | | | |

### Example 2: 5-methyl-1,3-benzenediacetonitrile (3)

3, 5-Bisbromomethyl toluene **2** (4.2 kg, 15 mol), 1.6 kg NaCN (33 mol, 2.2 meq) and ethanol (24 1) were mixed and heated at reflux for 2 hours. After the completion of the reaction, the mixture was concentrated and extracted into ethyl acetate. After removal of the solvent under reduced pressure crude 5-methyl-1,3-benzenediacetonitrile **3** was obtained, which was re-crystallised from 2-propanol to yield pure **3.** Yield 65 %.

### Example 3: α,α,α',α'-tetramethyl-5-methyl-1,3-benzene-diacetonitrile (4)

NaH (1.25 kg; 49.5mol, 5.5 meq) was added to a solution of 1.53 kg 5-methyl-1,3-benzenediacetonitrile **3** (9 mol) in 20 1 DMF. The mixture was stirred and cooled to about 10 °C. A solution of 2,8 1 methyl iodide (45 mol, 5 meq) in 7 1 DMF was added slowly into the cooled mixture. During the addition, the temperature of the reaction mixture was controlled such that 35 °C were not exceeded. After the addition, the mixture was stirred for about 4 hours at room temperature. To the stirred solution 25 1 of H₂O and 25 1 ethyl acetate were added. The mixture was stirred for about 10 minutes and then phase separated. To the aqueous layer another 25 1 of ethyl acetate were added. The mixture was stirred for about 10 minutes and then phase separated. The aqueous layer was discarded, and the organic layers were combined. The combined organic layers were washed with 25 1 H₂O three times. The organic layer was concentrated to dryness to obtain crude α,α,α',α'-tetramethyl-5-methyl-1,3-benzene-diacetonitrile **4.** The crude product was purified via crystallisation from 2-propanol to obtain purified **4** in a yield of 60 %.

### Example 4: α,α,α',α'-tetramethyl-5-bromomethyl-1,3-benzene-diacetonitrile (5)

To 1.13 kg of α,α,α',α'-tetramethyl-5-methyl-1,3-benzene-di-acetonitrile **4** (5 mol) in 6 1 of acetonitrile, 1.0 kg NBS (6 mol, 1.2 meq) and 0.125 kg AIBN (0.75 mol, 0.015 meq) were added. The mixture was stirred and kept at reflux for 3 hours.

The reaction progress was monitored by HPLC (column: RP-18, 4 x 250 mm, 5 µm (Merck, Lot L 552433); detection: UV = 210 nm; flow rate: 1 ml/min; mobile phase: acetonitrile/water = 1:1), the results of the analysis at the end of the reaction are shown in Table 2. After completion, the solvent was evaporated under reduced pressure. To the residue 20 1 of ethyl acetate and 20 1 of water were added. The mixture was stirred for about 10 minutes and then phases were separated. The organic layer was washed with water three times and concentrated to dryness. Crude α,α,α',α'-tetramethyl-5-bromomethyl-1,3-benzene-diacetonitrile **5** was obtained quantitatively with a purity of about 81 % and was used directly without further purification.

The above process was repeated but using cyclohexane and carbon tetrachloride, respectively, instead of acetonitrile as the solvent. The reaction mixtures were analysed by HPLC as described above. The results are also shown in Table 2.

**Table 2 Bromination of α,α,α',α'-tetramethyl-5-methyl-1,3-benzene-diacetonitrile 4**

| **solvent/ bromination agent** | **Product 5 [%]**^{**1)**} | **Unreacted Educt 4 [%]**^{**1)**} | **Side products [%]**^{**1)**} |
|---|---|---|---|
| CCl₄/ NBS²⁾ | 70.0 | 2.9 | 27.1 |
| Cyclohexane/ NBS | 64.4 | 29.7 | 3.8 |
| Acetonitile /NBS | 81.0 | 1.1 | 17.6 |

| | | | |
|---|---|---|---|
| ¹⁾ determined by HPLC | | | |
| ²⁾ comparative example | | | |

### Example 5: α,α,α',α'-tetramethyl-5-(1H-1,2,4-triazol-1-yl-methyl)-1,3-benzenediacetonitrile (anastrozole, 6)

To 1.5 kg of crude α,α,α',α'-tetramethyl-5-bromomethyl-1,3-benzene-diacetonitrile 5 (purity > 80%, about 3 mol) in 7.5 1 DMF 0.275 kg 1,2,4-triazole sodium (3 mol) were added. The mixture was stirred at room temperature for 1 hr. After completion, 10 1 ethyl acetate and 25 1 water were added. The mixture was stirred for about 10 minutes and then phase separated. To the aqueous layer 5 1 ethyl acetate were added. The mixture was stirred for about 10 minutes and then phase separated. The aqueous layer was discarded, and the organic layers were combined. The combined organic layers were washed with 10 1 water twice. The organic layer was concentrated to dryness to obtain the crude anastrozole **6** (90 %, purity > 60%). The crude product was purified by flash column chromatography (silica) using ethyl acetate as eluent (purity > 98%). Finally, the pre-purified anastrozole was re-crystallised from ethyl acetate/cyclohexane (1:2) to obtain **6** with a purity of more than 99.9%. Yield 34 %.

### Example 6: 3,5-bisbromomethyl toluene (2)

Mesitylene **1** (6 kg; 50 mol) was diluted in a 25-fold excess of cyclohexane (150 1). The solution was stirred at room temperature and bromine (16 kg, 100 mol, 2 meq) was added. After stirring for 3 hours at room temperature, the reaction mixture was analysed by HPLC (column: RP-18, 4 x 250 mm, 5 µm (Merck, Lot L 552433); detection: UV = 265 nm; flow rate: 1 ml/min; mobile phase: acetonitrile/water = 2:1), the results of the analysis are shown in Table 1 above. In order to isolate the product in pure form the reaction mixture was washed with 100 1 of water three times and extracted with ethyl acetate. The combined organic layers were concentrated to dryness under reduced pressure. The residue was crystallised from EtOH to obtain the purified 3,5-bisbromomethyl toluene **2.** Yield: 35 %.

## Claims

1. A process for the side-chain bromination of alkylbenzenes according to Formula (I) 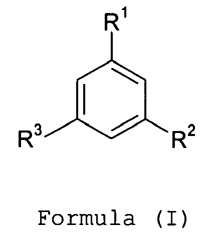 wherein
R¹ is C₁-C₆-alkyl;
R² is H or C₁-C₆-alkyl which can be unsubstituted or substituted by one or more cyano groups;
R³ is H or C₁-C₆-alkyl which can be unsubstituted or substituted by one or more cyano groups;
comprising the steps of
(1) dissolving the compound of Formula (I) in a solvent which is a non-aromatic non-halogenated hydrocarbon which can be substituted by one or more cyano groups; and
(2) adding a bromination agent selected from the group consisting of Br₂ and N-bromoimides to the solution.

2. The process of claim 1, further comprising
(3) agitating the mixture at a temperature within the range of from room temperature to reflux temperature.

3. The process of claim 1 or 2, wherein the solvent is selected from the group consisting of cyclohexane and acetonitrile.

4. The process of any one of the preceding claims further comprising the addition of a radical initiator.

5. The process of any one of the preceding claims, wherein the N-bromoimide is N-bromosuccinimide, N-bromophthalimide or N-bromoacetamide.

6. The process of any one of the preceding claims, wherein R¹ is C₁-C₃-alkyl, R² is C₁-C₃-alkyl, and R³ is C₁-C₃-alkyl.

7. The process of claim 6, wherein R¹, R² and R³ are each methyl.

8. The process of claim 6 or 7, wherein the solvent is cyclohexane.

9. The process of any one of claims 6 to 8, wherein the bromination agent is Br₂ or N-bromosuccinimide.

10. The process according to any one of the preceding claims, wherein the product of the bromination is 3,5-bisbromomethyl toluene **2.**

11. The process of any one of claims 1 to 5, wherein R¹ is C₁₋C₃-alkyl, R² is C₁-C₃-alkyl substituted by one cyano group, and R³ is C₁-C₃-alkyl substituted by one cyano group.

12. The process of claim 11, wherein the solvent is cyclohexane or acetonitrile.

13. The process of claim 11 or 12, wherein the bromination agent is N-bromosuccinimide.

14. The process of any one of claims 7 to 10, wherein the bromine residue(s) of the brominated product is/are substituted by cyano group(s) and the cyano substituted product is then subjected to the process of any one of claims 11 to 13.

15. The process of claim 14, wherein the bromine residue(s) are substituted by reaction with NaCN or KCN.

16. The process of claim 15, wherein the cyano substituted product is alkylated in α-position to the cyano group(s).

17. The process of claim 16, wherein the cyano substituted product is alkylated by reaction with NaH and CH₃I.

18. The process of any one of claims 11 to 17, wherein the product of the bromination is α,α,α',α'-tetramethyl-5-bromomethyl-1,3-benzene-diacetonitrile **5.**

19. The process of any one of claims 11 to 18, wherein the product of the process is further reacted with 1,2,4-triazole sodium.

20. The process of claim 19 giving α,α,α',α'-tetramethyl-5-(1H-1,2,4-triazol-1-ylmethyl)-1,3-benzenediacetonitrile **6** as the final product.
